# EUROPÄISCHE PATENTSCHRIFT

(11) **EP 4 661 797 B1**
(45) Veröffentlichungstag und Bekanntmachung des Hinweises auf die Patenterteilung: **02.12.2026**
(21) Anmeldenummer: 24708135.9
(22) Anmeldetag: 06.02.2024
(51) Int. Cl.: A61C 13/00, A61C 7/00, A61C 9/00

(54) **VERFAHREN UND ANALYSESYSTEM ZUR ERSTELLUNG EINES DIGITALEN 3D-MODELLS DES GEBISSES EINES PATIENTEN**
METHOD AND ANALYSIS SYSTEM FOR CREATING A DIGITAL 3D MODEL OF THE DENTITION OF A PATIENT
PROCÉDÉ ET SYSTÈME D'ANALYSE POUR ÉTABLIR UN MODÈLE 3D NUMÉRIQUE DE LA DENTITION D'UN PATIENT

(30) Priorität: 07.02.2023 DE 102023102973
(43) Veröffentlichungstag der Anmeldung: 17.12.2025
(73) Patentinhaber: Bite-Finder AG, 4410 Liestal (CH)
(72) Erfinder: ZÄUNER, Björn, 4410 Liestal (CH); ZÄUNER, Mike, 4410 Liestal (CH); HOFMANN, Markus, 4410 Liestal (CH)
(74) Vertreter: Tergau & Walkenhorst Intellectual Property GmbH
(86) Internationale Anmeldenummer: PCT/EP2024/052935
(87) Internationale Veröffentlichungsnummer: WO 2024/165572

(56) Entgegenhaltungen:
- DE-B3- 102017 124 580
- US-A1- 2002 150 859

## Beschreibung

Die Erfindung betrifft ein Verfahren zur Erstellung eines digitalen 3D-Modells des Gebisses eines Patienten. Sie bezieht sich weiter auf ein zur Durchführung des Verfahrens geeignetes Analysesystem, insbesondere zur Verwendung bei der Planung von Zahnersatz.

In diesem Zusammenhang wird auf die DE 10 2017 124580 B3 und die US 2002/150859 A1 verwiesen.

Bei der Planung von Zahnersatz, für die orthodontische Planung oder auch in sonstigen Bereichen der modernen Zahntechnik kann, insbesondere zur Verbesserung der Entscheidungsgrundlage oder Ausgangsbasis bei der Verwendung von CAD/CAM-Prozessen in der Zahnmedizin und Zahntechnik, die Aufbereitung und Analyse einer zahntechnischen Ausgangslage des Patienten oder auch eine Zwischen- oder Endkontrolle einer therapeutischen Versorgung anhand vorliegender oder bedarfsgerecht erfasster digitaler Daten erfolgen, die die Zahnsituation entsprechend wiedergeben.

In modernen dentalen Versorgungskonzepten wird beispielsweise üblicherweise die Anfertigung von Prothesen, wie beispielsweise Kronen, und/oder von implantatgetragenem Zahnersatz, Brücken oder dergleichen unter Rückgriff auf eine möglichst genaue Wiedergabe der Mundsituation des Patienten durchgeführt, um eine möglichst hohe Passgenauigkeit und damit zusätzlich zu den angestrebten medizinischen Effekten auch einen möglichst hohen Tragekomfort für den Patienten zu erreichen. Bei konventionellen Behandlungen war dazu bislang die Anfertigung einer so genannten Abformung üblich, mit der die Ist-Situation des Patientengebisses in einer Art Kit, einer Abformmasse oder einer anderen aushärtenden Substanz als Negativ-Abdruck erfasst wurde. Dieser kann sodann zur Anfertigung beispielsweise eines Gipsmodells mit Gips ausgegossen werden. Dieses Gipsmodell kann dann als Planungsgrundlage für die Anfertigung und auch die Einbringung von Zahnersatz herangezogen werden. Diese können dann, beispielsweise mittels extraoralem 3D-Scanner, digitalisiert werden, um nachfolgend mit digitalen Technologien (CAD/CAM) die zahntechnische Arbeit herzustellen.

In moderneren Konzepten erfolgt diese Aufnahme der Mundsituation des Patienten digital, wobei beispielsweise mittels so genannte Intra-Oralscanner dreidimensionale Patientendaten aufgenommen werden, die die Mundsituation des Patienten wiedergeben. Mittels dieser dreidimensionalen Daten können dann 3D-Modelle des Patientengebisses erstellt werden (beispielsweise als 3D-Druck), anhand derer dann mit digitalen Methoden, und somit deutlich schneller und kostengünstiger als bisher, die Versorgungsstrategie und der Zahnersatz für den Patienten geplant werden können. Insbesondere kann als Ergebnis einer solchen digitalen Planung der benötigte Zahnersatz unmittelbar anhand übertragbarer 3D-Daten automatisiert angefertigt werden.

Allerdings unterliegen diese digitalen Methoden derzeit einer gravierenden Einschränkung. Prinzipbedingt und im Hinblick auf die technischen Möglichkeiten und Grenzen der verwendeten Scanner zur Erfassung der 3D-Patientendaten im Patientenmund ist es nämlich nur möglich, den Oberkiefer des Patienten einerseits und den Unterkiefer des Patienten andererseits, jeweils getrennt voneinander, zu erfassen. Nach der Abtastung der Mundsituation, beispielsweise mittels eines Intra-Oralscanners, liegen die Daten somit lediglich in Form eines digitalen 3D-Modells des Oberkiefers des Patienten einerseits und eines digitalen 3D-Modells des Unterkiefers des Patienten vor. Für die ordnungsgemäße Wiedergabe der gesamten Mundsituation des Patienten müssen diese beiden Teilmodelle noch derart in Verbindung miteinander gebracht werden, dass sie der realen Mundsituation möglichst weitgehend entsprechend.

Diese Kombination des digitalen 3D-Modells des Oberkiefers mit dem digitalen 3D-Modell des Unterkiefers des Patienten wird als "Biss" bezeichnet. Der Biss ist die essentielle Ausgangslage, um alle weiteren Schritte in der Zahntechnik auf vernünftigem Niveau und mit einem für den Patienten akzeptablen Qualitätsniveau weiterzuführen. Die bisher hierzu verwendeten technischen Geräte, beispielsweise 3D-Scanner intraoral und extraoral, zur Erfassung von digitalen Zahnmodellen generieren nicht ausreichend genaue Bisse.

Die technische Ausgangsbasis sieht aktuell insbesondere die Digitalisierung von Einzelkiefern vor, und im zweiten Schritt von dem manuell ausgerichteten Biss. Daraufhin werden die Einzelkiefer-Scans mit dem Biss-Scan verglichen (gematcht), um Sie entsprechend auszurichten. Dabei wird üblicherweise eine Z-Achsen-Verschiebung genutzt, um eventuell auftretende Durchdringungen oder Überlappungen der beiden Kiefer zu beseitigen. Diese Vorgehensweise ist allerdings schon prinzipbedingt unbefriedigend, da der Kiefer sich nicht nur in z-Achse, sondern auch entlang der Kondylenbahn, verschieben lässt, so dass der genannte Ansatz unvollständig ist. Dies sorgt für hohe Fehleranfälligkeit in der nachfolgenden Prozesskette von CAD (Design) bis CAM (Produktion).

Der Erfindung liegt daher die Aufgabe zugrunde, ein Verfahren zur Erstellung eines digitalen 3D-Modells des Gebisses eines Patienten, insbesondere zur Verwendung bei der Planung von Zahnersatz, anzugeben, mit dem auf besonders zuverlässige und qualitativ hochwertige Weise unter Rückgriff auf vorliegende digitale 3D-Modelle sowohl des Oberkiefers als auch des Unterkiefers des Patienten ein vollständiges digitales 3D-Modell des Gebisses eines Patienten erstellt werden kann. Des Weiteren soll ein zur Durchführung des Verfahrens besonders geeignetes automatisches Analysesystem angegeben werden.

Bezüglich des Verfahrens wird diese Aufgabe erfindungsgemäß gelöst, indem in einer Recheneinheit für eine Vielzahl von jeweils eine Kombination eines digitalen 3D-Modells des Oberkiefers mit einem digitalen 3D-Modell des Unterkiefers des Patienten umfassenden Gebissvarianten, die sich in der relativen Positionierung von Ober- und Unterkiefer zueinander voneinander unterscheiden, jeweils:
- das digitale 3D-Modell des Oberkiefers derart mit dem digitalen 3D-Modell des Unterkiefers des Patienten kombiniert wird, dass der Ober- und der Unterkiefer an einer Anzahl von Kontaktstellen aufeinander aufliegen, ohne dass eine räumliche Überlappung von Teilbereichen des Ober- und Unterkiefers miteinander (in der Praxis oft auch als "Durchdringung" bezeichnet) vorliegt, und
- die Kontaktfläche zwischen Oberkiefer und Unterkiefer ermittelt wird,
wobei wobei unter Berücksichtigung der Kontaktfläche eine optimierte Gebissvariante als 3D-Modell des Gebisses ausgewählt wird.

Die Erfindung geht dabei von der Überlegung aus, dass für das aus den beiden Komponenten Oberkiefer und Unterkiefer zusammengesetzte Gebissmodell dann von eine besonders hohen "Passgenauigkeit" und damit möglichst realitätstreuer Wiedergabe der tatsächlichen Mundsituation des Patienten ausgegangen werden kann, wenn die beiden Komponenten Oberkiefer und Unterkiefer möglichst weitgehend aneinander anliegen. Die Kontaktfläche, über die eine Berührung dieser Komponenten miteinander vorliegt, wird daher gemäß einem Aspekt der Erfindung als besonders geeignetes Kriterium für die Passgenauigkeit angesehen und dementsprechend berücksichtigt.

Gemäß einem als eigenständig erfinderisch angesehenen Aspekt der Erfindung kann für die Bestimmung der Passgenauigkeit alternativ oder zusätzlich auch die Haftreibung zugrunde gelegt werden, die sich bei einer angedachten Verschiebung der Komponenten Oberkiefer und Unterkiefer relativ zueinander ergibt. Diese wird im Wesentlichen durch die Kontaktfläche beschrieben, wobei allerdings für eine verfeinerte Auswertung abhängig vom Neigungswinkel eines jeweiligen Flächenelements relativ zur angedachten Verschiebungsrichtung der Komponenten gegeneinander eine individualisierte Wichtung der Beiträge des jeweiligen Flächenelements zur Haftreibung vorgenommen werden kann. Flächenelemente mit vergleichsweise starker Neigung relativ zur Verschiebungsrichtung können dabei beispielsweise mit einem erhöhten Beitrag zur Haftreibung gewichtet werden, da sie allein schon formbedingt der angedachten Verschiebung mehr Widerstand entgegensetzen als vergleichsweise flach ausgerichtete Flächenelemente.

Gemäß einem als eigenständig erfinderisch angesehenen Aspekt der Erfindung kann dabei eine Mehrzahl von einer zeitlichen Abfolge innerhalb einer Kaubewegung entsprechenden Gebissvarianten für die Auswahl des 3D-Modells des Gebisses berücksichtigt werden. Damit soll insbesondere erreicht werden, dass nicht nur statische, sondern auch dynamische, also während der Kaubewegung auftretende, Aspekte bei der Auswahl des 3D-Modells berücksichtigt werden. Wie sich nämlich überraschend herausgestellt hat, können gerade diese für die Versorgung des Patienten und dessen gefühltem Tragekomfort einer angefertigten Prothetik besonders wichtig sein.

Gemäß einem weiteren Aspekt der Erfindung können dabei unterschiedliche Beiträge zur Reibung zwischen den Zähnen geeignet berücksichtigt werden. Die Beiträge zur Reibung können beispielsweise umfassen: Kontaktreibung (Zahnschmelz), Flüssigkeitsreibung (Speichel), sowie formbedingte Verzahnungseffekte.

Vorteilhafterweise werden bei der Bewertung der Gebissvarianten auch solche berücksichtigt, die sich in der Neigung des Oberkiefers relativ zum Unterkiefer voneinander unterscheiden. Damit kann den sechs Raumfreiheitsgraden bei der Kombination der beiden Elemente Oberkiefer und Unterkiefer Rechnung getragen werden.

Gemäß einem Aspekt der Erfindung kann insbesondere diejenige Gebissvariante als 3D-Modell des Gebisses ausgewählt werden, für die die Kontaktfläche von Ober und Unterkiefer einen Maximalwert annimmt.

Besonders bevorzugt erfolgt die Erstellung der Gebissvarianten und/oder Ermittlung der auszuwählenden Gebissvariante mittels künstlicher Intelligenz. Dabei liegt die Überlegung zugrunde, dass die Bestimmung eines optimalen Einrastpunktes oder der optimalen Kombination der Ober- und Unterkiefermodelle miteinander aufgrund der komplizierten Reibungsverhältnis zwischen den Zähnen (Haftreibung, Flüssigkeitsreibung, Verzahnungseffekte etc.) kaum durch eine klassische Modellierung abzubilden ist und deshalb gemäß einem Aspekt der Erfindung vorgesehen ist, ein Neuronales Netzwerk, anhand von Beispielen, die Ermittlung der erforderlichen Reibzustände anlernen zu lassen.

Unter Nutzung künstlicher Intelligenz kann dabei gemäß in jeweils als eigenständig erfinderisch angesehenen Aspekten der Erfindung in einem Voranalyseschritt ein Okklusionsalgorithmus verschiedene moderne Techniken aus der Computergrafik auf die 3D-Modelle der Zahnstrukturen anwenden. Dazu können die Verwendung von Filtern zur Erkennung von Extrema und Kanten sowie die selektive Glättung mit Hilfe partieller Differentialgleichungen zur Erkennung relevanter Zahnbereiche verwendet werden. Zusätzlich können die Hauptkomponentenanalyse und die Mustererkennung eingesetzt werden, um relevante Geometrien zu identifizieren. Das Hauptziel der Voranalyse besteht vorteilhafterweise darin, so viele Informationen wie möglich über Muster, Kanten und geometrische Strukturen aus dem 3D-Modell zu extrahieren.

Anhand der in der Voranalyse gewonnenen Informationen kann gemäß einem Aspekt der Erfindung in einem zweiten Schritt ein auf Hyperwürfeln basierender Algorithmus für die Neuroevolution von Augmentierungstopologien trainiert werden. Dabei werden beispielsweise 1875 Trainingsbeispiele verwendet, um dem Algorithmus beizubringen, wie er die Okklusion genau berechnen kann. Das Schlüsselkonzept in diesem zweiten Schritt besteht gemäß einem Aspekt der Erfindung darin, ein neuronales Netz zu trainieren, um die korrekte Okklusion zu bestimmen. Während des gesamten Lernprozesses wird das neuronale Netz vorteilafterweise erweitert, so dass es seine Topologie anpassen kann. Diese Flexibilität ermöglicht es ihm, die während der Voranalyse gewonnenen Informationen effektiv zu nutzen und zu kombinieren, wodurch die Freiheit des neuronalen Netzes beim Lernen und Berechnen der korrekten Okklusion erhöht wird.

Verschiedene Filter und Techniken aus dem Bereich der Computergrafik können gemäß Aspekten der Erfindung verwendet werden, um relevante Informationen aus 3D-Modellen zu extrahieren. Anschließend wird vorteilhafterweise ein spezielles neuronales Netz trainiert, um die Okklusion auf der Grundlage der extrahierten Informationen zu berechnen.

In Erweiterung der Kl-gesteuerten Bewertung der statischen Okklusion kann die dynamische Okklusion durch Kl-gestützte Analyse der Zahnfacettenformen bestimmt werden. Auch hier wird vorteilhafterweise ein auf Hyperwürfeln basierender Algorithmus zur Neuroevolution von Augmentierungstopologien eingesetzt. Zusätzlich können mögliche Kiefergelenkbewegungen durch differentialalgebraische Ungleichungssysteme beschrieben werden. Aus Sicht der Bayes'schen Statistik bilden die differentiellen algebraischen Ungleichungssysteme eine Prioritätsverteilung. Diese Verteilung wird gemäß einem Aspekt der Erfindung von der KI mit Hilfe der aus der Zahnform abgeleiteten Informationen immer weiter verfeinert und nähert sich so schrittweise den Grenzen des informationstheoretisch Machbaren.

Bezüglich des automatischen Analysesystems zur Verwendung bei der Planung von Zahnersatz wird die genannte Aufgabe gelöst, indem es Folgendes umfasst:
eine erste Rechenvorrichtung, die einen Speicher umfasst, der nicht-transitorische Befehle speichert, die, wenn sie von einem oder mehreren Prozessoren der ersten Rechenvorrichtung ausgeführt werden, die erste Rechenvorrichtung veranlassen:
von einer zweiten Rechenvorrichtung, die einen Massenspeicher umfasst, in dem ein digitales 3D-Modell des Oberkiefers eines Patienten und ein digitales 3D-Modell des Unterkiefers des Patienten hinterlegt sind, die digitalen 3D-Modelle des Oberkiefers und des Unterkiefers auszulesen,
für eine Vielzahl von jeweils eine Kombination des digitalen 3D-Modells des Oberkiefers mit dem digitalen 3D-Modell des Unterkiefers des Patienten umfassenden Gebissvarianten, die sich in der relativen Positionierung von Ober- und Unterkiefer zueinander voneinander unterscheiden, jeweils:
   - das digitale 3D-Modell des Oberkiefers mit dem digitalen 3D-Modell des Unterkiefers des Patienten derart miteinander zu kombinieren, dass der Ober- und der Unterkiefer an einer Anzahl von Kontaktstellen aufeinander aufliegen, ohne dass eine räumliche Überlappung von Teilbereichen des Ober- und Unterkiefers miteinander vorliegt, und
   - die Kontaktfläche zwischen Oberkiefer und Unterkiefer zu ermitteln,
sowie unter Berücksichtigung der Kontaktfläche eine optimierte Gebissvariante als 3D-Modell des Gebisses auszuwählen.

Vorzugsweise veranlassen die nicht-transitorischen Befehle, wenn sie von einem oder mehreren Prozessoren der ersten Rechenvorrichtung ausgeführt werden, die erste Rechenvorrichtung, Gebissvarianten zu berücksichtigen, die sich in der Neigung des Oberkiefers relativ zum Unterkiefer voneinander unterscheiden.

Vorzugsweise veranlassen die nicht-transitorischen Befehle, wenn sie von einem oder mehreren Prozessoren der ersten Rechenvorrichtung ausgeführt werden, gemäß einem Aspekt der Erfindung die erste Rechenvorrichtung, diejenige Gebissvariante als 3D-Modell des Gebisses auszuwählen, für die die Kontaktfläche von Ober und Unterkiefer einen Maximalwert annimmt.

Insbesondere ist gemäß einem Aspekt der Erfindung ein Konzept vorgesehen, das vollautomatisch die Okklusionsfindung simuliert und einen möglichst perfekten Biss von zwei digitalen Zahnmodellen findet. Der zugrundeliegende Algorithmus verarbeitet vorzugsweise zwei digitale Modelle (für Ober- und Unterkiefer, in beliebiger Ausrichtung) und ermittelt den perfekten Biss durch Simulation von Kieferbewegungen. Zur Ausrichtung wird gemäß einem Aspekt der Erfindung ein selbstlernender Algorithmus genutzt, der auf Basis von vielen digitalen Modellen eine Grundausrichtung findet.

In einem Aspekt der Erfindung kann vorgesehen sein, die Modelle in einem 3D Bild (DVT, MRT, CT) patientenindividuell zu positionieren und eine auf Basis des vorhergehenden Algorithmus der statischen Okklusion errechneten Ergebnisses eine Gelenkpositionierung zu berechnen.

Gemäß einem als eigenständig erfinderisch angesehenen Aspekt kann das nach dem vorstehend beschriebenen Konzept ermittelte und ausgewählte 3D-Modell des Gebisses als Grundlage für die Anfertigung einer Zahnprothetik verwendet werden.

In vorteilhaften Ausgestaltungen können nach Belieben weitere Algorithmen einzeln oder in Kombination miteinander genutzt werden, die:
- Modelle reparieren
- Löcher schließen, Artefakte entfernen oder reduzieren
- Dateigröße verringern so gut wie verlustfrei durch einen proprietären Kompressionsalgorithmus (schnellere CAD Verarbeitung, Speichereffizienz,..)
- Sockeln / Scan to print
- Modelle werden wasserdicht geschlossen und der Dateiname eingraviert (+ausgerichtet) um sie direkt an einen 3D-Drucker zur Herstellung eines räumlichen Modells zu senden
- dynamische Okklusion
- eine Simulation aller möglichen Unterkieferbewegungen auf Basis der Bennett Winkel, Kondylenbahnneigung und Schlifffacetten durchführen. Dabei kann gemäß einem Aspekt der Erfindung auch die Bewegung ("ISS", Immediate Side Shift) berücksichtigt werden. Die Ausgabe kann vorteilhafterweise als XML-Datei erolgen, so dass eine Weiterverarbeitung in CAD Software ermöglicht ist, um Zahnersatz zu designen und die individuelle Bewegung dabei zu berücksichtigen
- Bisshebung (VDO) ist nach der Berechnung möglich durchzuführen, wobei gleichzeitig Kieferbewegungen berücksichtigt werden können. Dies kann ein wichtiger Bestandteil sein für das Design von Schienen, Tabletops oder sonstigen Restorationen/Apparaturen die eine Bisshebung erfordern/positiv beeinflussen.

Die mit der Erfindung erzielten Vorteile bestehen insbesondere in:
- Deutlicher Effizienzsteigerung im Labor und der Zahnarztpraxis (=weniger Zeitverlust)
- Deutlicher Reduzierung der Ausschussquote, führt zu geringeren Produktionskosten und geringerem Materialverbrauch
- Erhöhung des Automatisierungsgrades (=geringere Kosten, schnellere Lieferung)
- Gegenmaßnahme zu Fachkräftemangel, da Software leicht zu bedienen und automatische Qualitätskontrolle bietet
- Deutlicher Steigerung der Qualität (keine Mehrfachbesuche oder Folgeerkrankungen für Patienten beim Zahnarzt, weil der Zahnersatz nicht gepasst hat.)
- Fehlendes Puzzleteil für einen vollständigen digitalen Workflow (aktuell immer noch analoge Zwischenschritte im Labor mit manuellen Anpassungen)

Gemäß weiterführenden, jeweils als eigenständig erfinderisch angesehenen Aspekten kann das beschrieben Konzept ergänzt werden durch:
- Integration von 2D- und/oder 3D-Röntgenbildern betreffed die Zahnsituation des Patienten. Damit können insbesondere für die Verwendung in der Künstlichen Intelligenz bereitgehaltenen Informationen angereichert und ergänzt werden, so dass die Ergebnisse weiter verbessert werden können
- Integration von klinischen Fotos oder 3D Gesichtsscans, um daraus hinreichend genau die Gelenkposition zu bestimmen
- Nutzung von statischen und dynamischen Dateb, um finale CAD Designs als automatische Qualitätskontrolle zu überprüfen und mit einem Bewertungsmaßstab zu versehen, der dann weitere automatische oder manuelle Schritte einleitet.
- Anpassung der 3D Sockel durch Dynamik und Statik, um automatisch an einen physischen Artikulator anzudocken.
- Simulation von weiteren Kaubewegungen
- Berechnung der Bisskraft durch einen Algorithmus
- automatisches Einbinden von weiteren patientenbezogenen Daten oder Dateien (neue scans, scanbodyscan, ...)
- Erstellung eines Occlusionreports mit Vergleich der Kontaktpunkte (vorzugsweise Vorher/nachher)
- Vorgabe / Hinweise für das folgende Design
- automatische Optimierung der Kronenposition auf Basis der Kontakte und der Dynamik
- Simulation der Kronenbewegung und der Spanne im Unterkiefer durch die Dynamik
- "4D JawMovement over time". Simulation der Kieferbewegungen über mehrere Jahre zur Visulalisierung der Auswirkungen auf die Zahnsubstanz (Dies kann dem Patienten die Effekte von Knirschen über die Zeit zeigen und ihn für weitere Behandlungsschritte wie z.B. Knirscherschiene motivieren)
- Erzeugen einer gewünschten Interkuspidations-/Kontaktpunktverteilung und daraus abgeleitet ein Einschleifprotokoll (2D oder als 3D-Datei zur Fertigung) um die Frühkontakte aufzulösen, die diese Situation zum Status quo verhindern.
- Nutzung der gewohnten Kieferbewegungen aus der Zahnsituation oder Prothesensituation um Folgeprothesen zu entwickeln, die angepasst sind
- Tabletop design automatisch auf Basis der Situation und der Kieferbewegungen, um neue Bisslagen zu definieren.
- Qualitätskontrolle der Modelle (Scan von 3D Modellen und automatischer Vergleich und Errechnung eines Scorewertes mit dem 3D CAD-Modell)
- Monitoring (Vergleich und Handlungsempfehlung von Modellen über Zeit z.B. bei jährlichem Zahnarztbesuch. Hinweis auf Veränderungen Schlifffacetten etc.)

In weiteren vorteilhaften Aspekten der Erfindung kann, beispielsweise in der Art entsprechender Module oder integrierter Funktionalitäten, vorgesehen sein:
- ein Modul "Save-by-Transformation": Dies kann die Möglichkeit bieten, aus 3D-Formaten nur die Vertices auszulesen und diese mit Rotationsmatrizen zu manipulieren. Die Rotationsmatrizen sind in der Praxis genau die berechneten Transformationen, um die Modelle in Okklusion zu bringen. Die als eigenständig erfinderisch angesehene Idee hierbei ist, dass das 3D-Format nicht komplett neu gespeichert werden muss, da dabei Zusatzinformation wie Farbinformationen verloren gehen können. Außerdem ist es möglich, "anhängende" Modelle, wie Brücken etc., ebenfalls in die Okklusion zu transformieren.
- die Bereitstellung von Markierfunktionen im GUI ("graphical user interface"): dadurch kann es dem Nutzer ermöglicht werden anzugeben, ob gewisse Bereiche Kontakt haben oder ignoriert werden sollen. Der Nutzer hat dadurch die Möglichkeit, Einfluss auf die Okklusionsberechnung zu nehmen, beispielsweise kann er Zahnfleisch markieren und dadurch eine höhere Durchdringung im Zahnfleischbereich erlauben etc. (Zahnfleisch gibt nach, wenn man hineinbeißt)
- eine Einstellmöglichkeit für die Durchdringungstiefe: Der Nutzer hat damit die Möglichkeit, die maximale Durchdringungstiefe einzustellen und dadurch mehr/größere und weniger/kleinere Kontakte zu erzeugen.
- eine Möglichkeit, anhand der dynamischen Okklusionsdaten eine Bisshebung durchführen.
- eine Bereitstellung eines Reports (z. B. als Video), welcher die Vorher- mit der Nachhersituation anhand der Kontaktpunkte vergleicht und anhand von Indikatoren bewertet.

Insbesondere können verschiedene Okklusionsalgorithmen vorgesehen sein, zwischen denen der Nutzer auswählen kann:
- befindet sich das Modell bereits nahe der Okklusion, kann ein lokaler Algorithmus verwendet werden, der die erlaubte Bewegung des Unterkiefers bis zur Okklusionsposition einschränkt.
- befindet sich das Modell fern der Okklusion, kann ein globaler Algorithmus verwendet werden, der die erlaubte Bewegung nicht einschränkt.

Das beschrieben Konzept "Bite-Finder" ermöglicht somit das Scannen mittels Intraoralscanner von Ober- und Unterkierfer bei entsprechender Bezahnung ohne den sonst nötigen dritten Scan der Okklusion. "Bite-Finder" ist somit ein innovatives Konzept, das die Erstellung digitaler 3D-Modelle des Bisses eines Patienten vereinfacht und so Zahnarztpraxen und Labore verändert. Diese Technologie revolutioniert die Dentalbranche, indem sie eine effizientere, genauere und kostengünstigere Methode für die Bissanalyse, -anpassung und Behandlungsplanung bietet. Es umfasst gemäß Aspekten der Erfindung die Hauptkomonenten:

### Bissdatenerfassung:

"Bite-Finder" lädt digitale 3D-Modelle sowohl des Ober- als auch des Unterkiefers eines Patienten. Diese Datenerfassung kann mit verschiedenen Mitteln erfolgen, z. B. mit intraoralen Scannern oder 3D-Zahnbildtechnologien.

### Kl-gestützte Ausrichtung:

Ein als eigenständig erfinderisch angesehener Aspekt von "Bite-Finder" liegt in seinen Klgestützten Ausrichtungsfunktionen. Sobald die digitalen Modelle des Ober- und Unterkiefers vorliegen, nutzt das System hochentwickelte Algorithmen, um diese Modelle mit unvergleichlicher Präzision auszurichten. Im Gegensatz zu herkömmlichen Methoden, die sich ausschließlich auf die Anpassung der Z-Achse verlassen, berücksichtigt Bite-Finder verschiedene Freiheitsgrade, einschließlich Translation und Rotation, und stellt so sicher, dass Ober- und Unterkiefer nahtlos zusammenpassen.

### Okklusions- und Penetrationsanalyse:

"Bite-Finder" führt gemäß einem Aspekt der Erfindung eine eingehende Analyse der ausgerichteten Modelle durch, um Okklusions- und Penetrationsprobleme zu identifizieren. Durch umfassende Simulationen und Berechnungen ermittelt das System die optimale Bisslage, indem es die Kontaktpunkte zwischen den Zähnen anpasst. Dieser Prozess stellt sicher, dass der Biss des Patienten in perfekter Harmonie ist, was zu einem verbesserten Komfort und besseren Behandlungsergebnissen führt.

### Dynamische Biss-Simulation:

Zusätzlich zu den statischen Bissanpassungen bietet "Bite-Finder" in einem Aspekt der Erfindung die Möglichkeit der dynamischen Biss-Simulation. Er kann patientenspezifische Kieferbewegungen nachbilden, so dass Zahnärzte und Zahntechniker beurteilen können, wie der Biss bei realen Aktivitäten wie Kauen und Sprechen funktioniert. Diese Funktion erhöht die Präzision von restaurativen Behandlungen und der Konstruktion von Apparaturen.

### Automatisierte Modellverbesserung:

"Bite-Finder" geht vorteilhafterweise über Bisskorrekturen hinaus. Er kann Modelle reparieren, Löcher schließen, Artefakte entfernen, die Dateigröße mit Hilfe proprietärer Kompressionsalgorithmen reduzieren und sogar Modelle für den 3D-Druck vorbereiten. Diese Funktionen tragen zu einem strafferen und effizienteren Arbeitsablauf bei.

### Qualitätskontrolle und -sicherung:

Um hohe Qualitätsstandards aufrechtzuerhalten, verfügt "Bite-Finder" vorteilhafterweise über automatische Qualitätskontrollen. Dadurch wird sichergestellt, dass die Bissanpassungen und Modelle den industriellen und klinischen Standards entsprechen.

Darüber hinaus sind Prognosen über die Entwicklung der Schliffacetten bzw. des Verschleißes möglich.

Besondere Nutzen und Vorteile der Erfindung können insbesondere gesehen werden in:
Exaktheit: Die Kl-gesteuerte Ausrichtung und Analyse führt zu hochpräzisen Bisskorrekturen und reduziert das Risiko von Nachbesserungen und Fehlern.
Effizienz: Durch die Automatisierung komplexer Prozesse reduziert Bite-Finder den Zeitaufwand für die Bissanalyse und Behandlungsplanung erheblich.
Kosteneffizienz: Weniger manuelle Anpassungen, weniger Materialabfälle und ein effizienterer Arbeitsablauf führen zu Kosteneinsparungen für Zahnarztpraxen und Labore. Patientenkomfort: Dank der präzisen Bissanpassung erleben die Patienten mehr Komfort während der Behandlung.
Vielseitigkeit: "Bite-Finder" kann in verschiedenen zahnmedizinischen Fachbereichen eingesetzt werden, von der allgemeinen Zahnmedizin bis hin zur Kieferorthopädie und Prothetik.

Das gemäß einem Aspekt der Erfindung vorgesehene "Bite-Finder"-Konzept ist im Ergebnis eine bahnbrechende Innovation, die Effizienz, Präzision und Erschwinglichkeit in die Dentalbranche bringt. Durch die Kombination von digitaler Technologie, Kl-Algorithmen und 3D-Modellierung vereinfacht es den Prozess der Erstellung präziser 3D-Bissmodelle, was letztendlich die Qualität der Patientenversorgung und der zahnärztlichen Behandlungen verbessert. Mit "Bite-Finder" können Zahnärzte und Zahntechniker effektiver arbeiten und ihren Patienten hervorragende zahnmedizinische Lösungen anbieten.

### Prozess:

- Preview ansehen und an Labor senden zur Nutzung (und Zahlung)

Als erfindungsgemäß angesehen werden kann gemäß einem Aspekt der Erfindung:
Ein Verfahren zur Erstellung eines digitalen 3D-Modells des Bisses eines Patienten, umfassend:
a. Erfassen von digitalen 3D-Modellen des Ober- und Unterkiefers eines Patienten unter Verwendung einer oder mehrerer Abbildungsvorrichtungen.
b. Verwendung von Algorithmen der künstlichen Intelligenz zum Ausrichten der digitalen 3D-Modelle des Ober- und Unterkiefers unter Berücksichtigung von Translation, Rotation und Freiheitsgraden.
c. Durchführung einer Okklusions- und Penetrationsanalyse zur Bestimmung der optimalen Bisslage durch Anpassung der Kontaktpunkte zwischen den Zähnen.
d. Generierung einer dynamischen Biss-Simulation zur Nachbildung der patientenspezifischen Kieferbewegungen für die Bissanalyse.

Vorteilhafterweise kann dabei ergänzend vorgesehen sein:
Reparieren digitaler Modelle, Schließen von Löchern und Entfernen von Artefakten zur Verbesserung der Modellqualität und/oder
Reduzieren der Dateigrößen unter Verwendung eines proprietären Kompressionsalgorithmus bei gleichzeitiger Aufrechterhaltung der Modellintegrität.und/oder
Aspekte, wobei die dynamische Biss-Simulation die Nachbildung realer Aktivitäten wie Kauen und Sprechen umfasst, um die Funktionalität des Bisses zu bewerten.und/oder
die Automatisierung der Vorbereitung der digitalen Modelle für den 3D-Druck und/oder
automatisierte Qualitätskontrollprüfungen durchgeführt werden, um sicherzustellen, dass die Bissanpassungen und Modelle vordefinierten Qualitätsstandards entsprechen und/oder
ein Computerprogrammprodukt, das computerlesbare Befehle umfasst, die auf einem nicht-transitorischen computerlesbaren Medium gespeichert sind, um das vorstehend beschriebene Verfahren auszuführen und/oder
ein durch künstliche Intelligenz gesteuertes System zur Erstellung eines digitalen 3D-Modells des Bisses eines Patienten, umfassend
   a. Eine oder mehrere Abbildungsvorrichtungen zum Erfassen von digitalen 3D-Modellen des Ober- und Unterkiefers eines Patienten.
   b. Eine Verarbeitungseinheit, die so konfiguriert ist, dass sie die digitalen 3D-Modelle des Ober- und Unterkiefers unter Verwendung von Algorithmen der künstlichen Intelligenz unter Berücksichtigung von Translation, Rotation und Freiheitsgraden ausrichtet.
   c. Ein Analysemodul zur Durchführung von Okklusions- und Penetrationsanalysen zur Bestimmung der optimalen Bissausrichtung durch Anpassung der Kontaktpunkte zwischen den Zähnen.

Dieses kann gemäß Aspekten der Erfindung ergänzt sein durch:
ein dynamisches Simulationsmodul zur Erzeugung einer dynamischen Biss-Simulation, um patientenspezifische Kieferbewegungen für die Bissanalyse nachzubilden, und/oder
ein Qualitätskontrollmodul zur Automatisierung von Qualitätskontrollprüfungen, um sicherzustellen, dass Bissanpassungen und Modelle vordefinierten Qualitätsstandards entsprechen und/oder
ein Modul zum Reparieren digitaler Modelle, Schließen von Löchern und Entfernen von Artefakten zur Verbesserung der Modellqualität und/oder
ein Modul zur Reduzierung der Dateigröße unter Verwendung eines proprietären Kompressionsalgorithmus bei gleichzeitiger Aufrechterhaltung der Modellintegrität und/oder
einen Aspekt, wobei das dynamische Simulationsmodul die Möglichkeit bietet, reale Aktivitäten wie Kauen und Sprechen nachzubilden, um die Funktionalität des Bisses zu bewerten und/oder
ein Modul zur Automatisierung der Vorbereitung von digitalen Modellen für den 3D-Druck, und/oder
eine Benutzerschnittstelle für zahnärztliches Fachpersonal zur Interaktion mit dem System.

Weitere Vorteile der Erfindung können gesehen werden in:
1. Verbesserte Genauigkeit: Bite-Finder verwendet fortschrittliche Algorithmen der künstlichen Intelligenz (Kl), um hochpräzise Bissanpassungen zu gewährleisten und das Risiko von Fehlern und Neuanfertigungen zu reduzieren.
2. Effizienzgewinne: Das System verkürzt die für die Bissanalyse und die Behandlungsplanung benötigte Zeit erheblich und verbessert so die Effizienz des gesamten Arbeitsablaufs.
3. Kosteneinsparungen: Weniger manuelle Anpassungen, weniger Materialabfälle und eine verbesserte Effizienz der Arbeitsabläufe führen zu Kosteneinsparungen für Zahnarztpraxen und Labore.
4. Verbesserter Patientenkomfort: Präzise Bissanpassungen führen zu einem verbesserten Patientenkomfort während der Verfahren und der Behandlung.
5. Vielseitigkeit: Bite-Finder kann in verschiedenen zahnmedizinischen Fachbereichen eingesetzt werden, von der allgemeinen Zahnheilkunde bis hin zur Kieferorthopädie und Prothetik, was ihn zu einem vielseitigen Werkzeug für Zahnärzte macht.
6. Rationalisierter Arbeitsablauf: Das System automatisiert komplexe Prozesse wie die Modellreparatur, das Schließen von Löchern, die Entfernung von Artefakten und die Reduzierung der Dateigröße, was den Arbeitsablauf für Zahnärzte und Zahntechniker vereinfacht.
7. Automatisierte Modellvorbereitung: Bite-Finder automatisiert die Vorbereitung digitaler Modelle für den 3D-Druck, was Zeit spart und den Bedarf an manuellen Eingriffen reduziert.
8. Qualitätskontrolle: Automatische Qualitätskontrollen stellen sicher, dass die Bissanpassungen und Modelle den industriellen und klinischen Standards entsprechen, wodurch die Wahrscheinlichkeit von suboptimalen Ergebnissen verringert wird.
9. Fehlerreduzierung: Durch die Minimierung manueller Eingriffe und die Abhängigkeit von menschlichem Urteilsvermögen reduziert Bite-Finder das Potenzial für menschliche Fehler, was zu konsistenteren Ergebnissen führt.
10. Kosteneffiziente CAD/CAM-Verarbeitung: Der systemeigene Komprimierungsalgorithmus reduziert die Dateigrößen ohne Einbußen bei der Modellqualität und ermöglicht so eine kosteneffizientere CAD/CAM-Verarbeitung.
11. Antworten auf die Herausforderungen der Branche: Bite-Finder geht auf bestehende Herausforderungen in der Dentalindustrie ein, wie z.B. den Bedarf an genauerer Bissmodellierung und die Nachfrage nach Automatisierung, um dem Arbeitskräftemangel entgegenzuwirken.
12. Verbesserte Behandlungsplanung: Die dynamische Biss-Simulationsfunktion ermöglicht es Zahnärzten, die Bissfunktionalität während realer Aktivitäten wie Kauen und Sprechen zu bewerten, was zu einer verbesserten Behandlungsplanung führt.
13. Patientenzufriedenheit: Präzise Bissanpassungen verbessern die Passform und den Komfort von Zahnspangen, was zu einer höheren Patientenzufriedenheit und weniger Beschwerden nach der Behandlung führt.
14. Rückmeldung in Echtzeit: Zahnärzte können Echtzeit-Feedback zu Bissanpassungen und Behandlungsplänen erhalten, was schnelle Anpassungen und Verbesserungen ermöglicht.
15. Vielseitige Anwendungen: Bite-Finder kann für verschiedene zahnärztliche Verfahren verwendet werden, einschließlich restaurativer Behandlungen, Kieferorthopädie, Implantologie und mehr, was ihn zu einem wertvollen Werkzeug in einer Reihe von Zahnarztpraxen macht.
16. Integrationsfähigkeiten: Das System kann mit anderen zahnmedizinischen Technologien wie 2D- und 3D-Bildgebung, klinischen Fotos und Gesichtsscans integriert werden, um Informationen anzureichern und Ergebnisse zu verbessern.
17. Potenzial für weitere Innovationen: Der modulare Aufbau von Bite-Finder ermöglicht die Integration zusätzlicher Algorithmen und Funktionen, was den Weg für weitere Innovationen im Dentalbereich ebnen kann.

Bevorzugte Anwendungsfelder und Einsatzgebiete können gesehen werden in:

### 1. Prothetik:

Präzise Bisskorrekturen für die Anfertigung von Kronen, Brücken und Prothesen.

Bewertung und Korrektur von okklusalen Diskrepanzen bei prothetischen Behandlungen.

### 2. Kieferorthopädie:

Beurteilung der Bissverhältnisse in kieferorthopädischen Fällen.

Simulation von Kieferbewegungen zur Planung kieferorthopädischer Behandlungen.

### 3. Implantologie:

Analyse der okklusalen Passung für implantatgetragene Versorgungen.

Sicherstellung einer optimalen Bisslage für die Implantatplanung.

### 4. Restaurative Zahnheilkunde:

Bissanalyse für restaurative Verfahren, wie Füllungen und Veneers.

Überprüfung des okklusalen Sitzes von Restaurationsmaterialien.

### 5. Okklusionsanalyse:

Umfassende Okklusionsanalyse zur Feststellung von Bissdiskrepanzen.

Erkennung und Korrektur von okklusalen Interferenzen.

### 6. Temporomandibuläre Gelenkstörungen (TMJ):

Bewertung von Kieferbewegungen und Bissverhältnissen bei Kiefergelenksbeschwerden.

Planung von Behandlungen zur Behebung von Kiefergelenksproblemen.

### 7. Behandlungsplanung:

Simulation der Bissfunktionalität während der Behandlungsplanung.

Optimierung von Behandlungsplänen auf der Grundlage der dynamischen Bissanalyse.

### 8. Beurteilung nach der Behandlung:

Überprüfung der Bissgenauigkeit und des Komforts nach zahnärztlichen Behandlungen.

Anpassungen und Verfeinerungen auf der Grundlage der Bissanalyse nach der Behandlung.

### 9. Prothetische Wiederherstellung:

Bite-Finder kann verwendet werden, um den Sitz und den Komfort verschiedener prothetischer Versorgungen zu optimieren, einschließlich Vollbogenrekonstruktionen und Teilprothesen.

### 10. Herausnehmbare Apparate:

Analyse der Bissverhältnisse bei herausnehmbaren Apparaturen wie Teilprothesen.

Sicherstellung eines komfortablen Sitzes für Patienten mit herausnehmbaren Zahnersatz.

### 11. Qualitätskontrolle:

Laufende Qualitätskontrollen in zahntechnischen Labors, um die Genauigkeit der digitalen Modelle zu überprüfen.

Identifizierung und Korrektur von Problemen in digitalen Modellen, bevor sie zu klinischen Problemen führen.

### 12. Zahnärztliche Ausbildung:

Schulung und Ausbildung von Zahnmedizinstudenten und Fachleuten in der Bissanalyse und -anpassung.

Simulation und Lehre von bissbezogenen Konzepten in einer virtuellen Umgebung.

### 13. Integration mit CAD/CAM:

Integration mit computergestütztem Design und computergestützter Fertigung (CAD/CAM) für einen nahtlosen digitalen Arbeitsablauf in Dentallabors.

### 14. Zusammenarbeit mit anderen Fachleuten:

Erleichterung der Zusammenarbeit zwischen Zahnärzten, z. B. Kieferorthopäden,

Prothetikern und Kieferchirurgen, durch Bereitstellung einer gemeinsamen digitalen Plattform für die Bissanalyse.

### 15. Forschung und Entwicklung:

Unterstützung der zahnmedizinischen Forschung und Entwicklung durch Bereitstellung präziser Bissmodellierungs- und Analysefunktionen für Studien und Experimente.

### 16. Posttraumatische Fälle:

Beurteilung und Korrektur von Bissproblemen infolge eines Zahntraumas.

Wiederherstellung der Bissfunktion und Ästhetik in posttraumatischen Fällen.

### 17. Pädiatrische Zahnheilkunde:

Analyse und Einstellung des Bisses bei pädiatrischen Patienten für verschiedene zahnärztliche Verfahren.

Sicherstellung der richtigen Okklusion und Ausrichtung bei Kindern im Wachstum.

### 18. Multidisziplinäre Fälle:

Einsatz bei komplexen multidisziplinären Fällen, bei denen mehrere Zahnärzte an der Behandlungsplanung und -durchführung beteiligt sind.

### 19. Kontinuierliche Überwachung:

Kontinuierliche Bissüberwachung bei Patienten mit Zahnersatz, um langfristigen Komfort und Wirksamkeit zu gewährleisten.

## Patentansprüche

1. Verfahren zur Erstellung eines digitalen 3D-Modells des Gebisses eines Patienten, bei dem in einer Recheneinheit für eine Vielzahl von jeweils eine Kombination eines digitalen 3D-Modells des Oberkiefers mit einem digitalen 3D-Modell des Unterkiefers des Patienten umfassenden Gebissvarianten, die sich in der relativen Positionierung von Ober- und Unterkiefer zueinander voneinander unterscheiden, jeweils:
- das digitale 3D-Modell des Oberkiefers mit dem digitalen 3D-Modell des Unterkiefers des Patienten derart miteinander kombiniert wird, dass der Ober- und der Unterkiefer an einer Anzahl von Kontaktstellen aufeinander aufliegen, ohne dass eine räumliche Überlappung von Teilbereichen des Ober- und Unterkiefers miteinander vorliegt, und
- die Kontaktfläche zwischen Oberkiefer und Unterkiefer ermittelt wird,
wobei unter Berücksichtigung der Kontaktfläche eine optimierte Gebissvariante als 3D-Modell des Gebisses ausgewählt wird.

2. Verfahren nach Anspruch 1, bei dem Gebissvarianten berücksichtigt werden, die sich in der Neigung des Oberkiefers relativ zum Unterkiefer voneinander unterscheiden.

3. Verfahren nach Anspruch 1 oder 2, bei dem diejenige Gebissvariante ausgewählt wird, für die die Kontaktfläche von Ober- und Unterkiefer einen Maximalwert annimmt.

4. Verfahren nach einem der Ansprüche 1 bis 3, bei dem eine Mehrzahl von einer zeitlichen Abfolge innerhalb einer Kaubewegung entsprechenden Gebissvarianten für die Auswahl des 3D-Modells des Gebisses berücksichtigt wird.

5. Verfahren nach einem der Ansprüche 1 bis 4, bei dem die Erstellung der Gebissvarianten und/oder Ermittlung der auszuwählenden Gebissvariante mittels künstlicher Intelligenz erfolgt.

6. Automatisches Analysesystem zur Durchführung des Verfahrens nach einem der Ansprüche 1 bis 5, das Folgendes umfasst:
eine erste Rechenvorrichtung, die einen Speicher umfasst, der nicht-transitorische Befehle speichert, die, wenn sie von einem oder mehreren Prozessoren der ersten Rechenvorrichtung ausgeführt werden, die erste Rechenvorrichtung veranlassen: von einer zweiten Rechenvorrichtung, die einen Massenspeicher umfasst, in dem ein digitales 3D-Modell des Oberkiefers eines Patienten und ein digitales 3D-Modell des Unterkiefers des Patienten hinterlegt sind, die digitalen 3D-Modelle des Oberkiefers und des Unterkiefers auszulesen,
für eine Vielzahl von jeweils eine Kombination des digitalen 3D-Modells des Oberkiefers mit dem digitalen 3D-Modell des Unterkiefers des Patienten umfassenden Gebissvarianten, die sich in der relativen Positionierung von Ober- und Unterkiefer zueinander voneinander unterscheiden, jeweils:
- das digitale 3D-Modell des Oberkiefers mit dem digitalen 3D-Modell des Unterkiefers des Patienten derart miteinander zu kombinieren, dass der Ober- und der Unterkiefer an einer Anzahl von Kontaktstellen aufeinander aufliegen, ohne dass eine räumliche Überlappung von Teilbereichen des Ober- und Unterkiefers miteinander vorliegt, und
- die Kontaktfläche zwischen Oberkiefer und Unterkiefer zu ermitteln,
sowie unter Berücksichtigung der Kontaktfläche eine optimierte Gebissvariante als 3D-Modell des Gebisses auszuwählen.

7. System nach Anspruch 6, bei dem die nicht-transitorischen Befehle, wenn sie von einem oder mehreren Prozessoren der ersten Rechenvorrichtung ausgeführt werden, die erste Rechenvorrichtung veranlassen, diejenige Gebissvariante als 3D-Modell des Gebisses auszuwählen, für die die Kontaktfläche von Ober- und Unterkiefer einen Maximalwert annimmt.

8. System nach Anspruch 6 oder 7, bei dem die nicht-transitorischen Befehle, wenn sie von einem oder mehreren Prozessoren der ersten Rechenvorrichtung ausgeführt werden, die erste Rechenvorrichtung veranlassen, Gebissvarianten zu berücksichtigen, die sich in der Neigung des Oberkiefers relativ zum Unterkiefer voneinander unterscheiden.

9. System nach einem der Ansprüche 6 bis 8, bei dem die nicht-transitorischen Befehle, wenn sie von einem oder mehreren Prozessoren der ersten Rechenvorrichtung ausgeführt werden, die erste Rechenvorrichtung veranlassen, eine Mehrzahl von einer zeitlichen Abfolge innerhalb einer Kaubewegung entsprechenden Gebissvarianten für die Auswahl des 3D-Modells des Gebisses zu berücksichtigen.

## Claims

1. A method for creating a digital 3D model of a patient's dentition, in which, in a computing unit, for a plurality of dentition variants, each of which comprises a combination of a digital 3D model of the upper jaw and a digital 3D model of the lower jaw of the patient, and which differ from one another in the relative positioning of the upper and lower jaws, the following are performed in each case:
- the digital 3D model of the upper jaw is combined with the digital 3D model of the patient's lower jaw, in such a way that the upper and lower jaws rest on each other at a number of contact points without any spatial overlap of partial regions of the upper and lower jaws, and
- the contact surface between the upper and lower jaw is determined, wherein, taking into account the contact surface, an optimized dentition variant is selected as 3D model of the dentition.

2. The method according to claim 1, in which dentition variants are taken into account that differ from one another in the inclination of the upper jaw relative to the lower jaw.

3. The method according to claim 1 or 2, in which the dentition variant is selected for which the contact surface between the upper and lower jaw assumes a maximum value.

4. The method according to any one of claims 1 to 3, in which a plurality of dentition variants corresponding to a temporal sequence within a chewing movement are taken into account for the selection of the 3D model of the dentition.

5. The method according to any one of claims 1 to 4, in which the creation of the dentition variants and/or determination of the dentition variant to be selected is carried out by means of artificial intelligence.

6. An automatic analysis system for carrying out the method according to any one of claims 1 to 5, comprising the following:
a first computing device comprising a memory that stores non-transitory instructions which, when executed by one or more processors of the first computing device, cause the first computing device:
to read out the digital 3D models of the upper jaw and the lower jaw from a second computing device comprising a mass storage device in which a digital 3D model of the upper jaw of a patient and a digital 3D model of the lower jaw of the patient are stored,
for a plurality of dentition variants, which each comprise a combination of the digital 3D model of the upper jaw with the digital 3D model of the lower jaw of the patient, and which differ from one another in the relative positioning of the upper and lower jaws with respect to one another, to perform the following in each case:
- to combine the digital 3D model of the upper jaw with the digital 3D model of the patient's lower jaw, in such a way that the upper and lower jaws rest on each other at a number of contact points without any spatial overlap of partial regions of the upper and lower jaws, and
- to determine the contact surface between the upper and lower jaw, and, taking into account the contact surface, to select an optimized dentition variant as a 3D model of the dentition.

7. The system according to claim 6, in which the non-transitory commands, when executed by one or more processors of the first computing device, cause the first computing device to select the dentition variant as a 3D model of the dentition for which the contact surface of the upper and lower jaws assumes a maximum value.

8. The system according to claim 6 or 7, in which the non-transitory commands, when executed by one or more processors of the first computing device, cause the first computing device to take into account dentition variants that differ from each other in the inclination of the upper jaw relative to the lower jaw.

9. The system according to any one of claims 6 to 8, in which the non-transitory commands, when executed by one or more processors of the first computing device, cause the first computing device to take into account a plurality of dentition variants corresponding to a temporal sequence within a chewing movement for the selection of the 3D model of the dentition.

## Revendications

1. Procédé pour établir un modèle tridimensionnel numérique de la dentition d'un patient, pour lequel dans une unité de calcul pour une pluralité de variantes de dentition comprenant respectivement une combinaison d'un modèle tridimensionnel numérique de la mâchoire supérieure avec un modèle tridimensionnel numérique de la mâchoire inférieure du patient, qui se différencient les unes des autres dans le positionnement relatif de la mâchoire supérieure et de la mâchoire inférieure :
- le modèle tridimensionnel numérique de la mâchoire supérieure est combiné ensemble avec le modèle tridimensionnel numérique de la mâchoire inférieure du patient de telle manière que la mâchoire supérieure et la mâchoire inférieure reposent l'une sur l'autre sur un nombre de points de contact sans qu'il y ait un chevauchement spatial des parties de zone de la mâchoire supérieure et de la mâchoire inférieure, et
- la surface de contact est déterminée entre la mâchoire supérieure et la mâchoire inférieure, sachant qu'une variante de dentition optimisée est choisie en tant que modèle tridimensionnel de la dentition en tenant compte de la surface de contact.

2. Procédé selon la revendication 1, pour lequel des variantes de dentition sont prises en considération qui se différencient l'une de l'autre dans l'inclinaison de la mâchoire supérieure par rapport à la mâchoire inférieure.

3. Procédé selon la revendication 1 ou 2, pour lequel la variante de dentition qui est choisie adopte une valeur maximale pour la surface de contact de la mâchoire supérieure et de la mâchoire inférieure.

4. Procédé selon l'une quelconque des revendications 1 à 3, pour lequel une pluralité de variantes de dentition correspondant à un ordre chronologique à l'intérieur d'un mouvement de mastication est pris en considération pour le choix du modèle tridimensionnel de la dentition.

5. Procédé selon l'une quelconque des revendications 1 à 4, pour lequel l'établissement des variantes de dentition et/ou la détermination de la variante de dentition à sélectionner a lieu au moyen de l'intelligence artificielle.

6. Système d'analyse automatique pour l'exécution du procédé selon l'une quelconque des revendications 1 à 5, qui comprend les éléments suivants :
un premier dispositif de calcul, qui comprend une mémoire, qui mémorise les ordres non transitoires, qui, lorsqu'ils sont exécutés par un ou plusieurs processeurs du premier dispositif de calcul, ordonnent au premier dispositif de calcul :
de sélectionner les modèles tridimensionnels numériques de la mâchoire supérieure et de la mâchoire inférieure par un deuxième dispositif de calcul, qui comprend une mémoire de masse dans laquelle sont mémorisés un modèle tridimensionnel numérique de la mâchoire supérieure d'un patient et un modèle tridimensionnel numérique de la mâchoire inférieure du patient,
pour une pluralité de variantes de dentition comprenant respectivement une combinaison du modèle tridimensionnel numérique de la mâchoire supérieure avec le modèle tridimensionnel numérique de la mâchoire inférieure du patient, qui se différencient l'une de l'autre dans le positionnement relatif de la mâchoire supérieure et de la mâchoire inférieure l'une par rapport à l'autre :
- de combiner respectivement entre eux le modèle tridimensionnel numérique de la mâchoire supérieure avec le modèle tridimensionnel numérique de la mâchoire inférieure du patient de telle manière que la mâchoire supérieure et la mâchoire inférieure reposent l'une sur l'autre sur un nombre de points de contact sans qu'il y ait un chevauchement spatial des parties de zone de la mâchoire supérieure et de la mâchoire inférieure entre elles, et
- de déterminer la surface de contact entre la mâchoire supérieure et la mâchoire inférieure, ainsi que de choisir une variante de dentition optimisée en tant que modèle tridimensionnel de la dentition en prenant en considération la surface de contact.

7. Système selon la revendication 6, pour lequel les ordres non transitoires, lorsqu'ils sont exécutés par un ou plusieurs processeurs du premier dispositif de calcul, qui ordonnent au premier dispositif de calcul de sélectionner la variante de dentition en tant que modèle tridimensionnel de la dentition pour laquelle la surface de contact de la mâchoire supérieure et de la mâchoire inférieure adopte une valeur maximale.

8. Système selon la revendication 6 ou 7, pour lequel les ordres non transitoires, lorsqu'ils sont exécutés par un ou plusieurs processeurs du premier dispositif de calcul, qui ordonnent au premier dispositif de calcul de prendre en considération des variantes de dentition, qui se différencient l'une de l'autre dans l'inclinaison de la mâchoire supérieure par rapport à la mâchoire inférieure.

9. Système selon l'une quelconque des revendications 6 à 8, pour lequel les ordres non transitoires, lorsqu'ils sont exécutés par un ou plusieurs processeurs du premier dispositif de calcul, ordonnent au premier dispositif de calcul de prendre en considération une pluralité de variantes de dentition correspondant à un ordre chronologique à l'intérieur d'un mouvement de mastication pour le choix du modèle tridimensionnel de dentition.
